# EUROPEAN PATENT APPLICATION

(11) **EP 2 080 759 A1**
(43) Date of publication of application: **22.07.2009**
(21) Application number: 07827886.8
(22) Date of filing: 12.10.2007
(51) Int. Cl.: C07D 401/14, A61K 31/4545, A61P 7/06, A61P 43/00

(54) **GAMA-GLOBIN INDUCER**

(30) Priority: 13.10.2006 JP 2006279893
(71) Applicant: Kowa Company, Ltd., Naka-ku Nagoya-shi, Aichi-ken 460-8625 (JP)
(72) Inventor: YAMABI, Masaki, Higashimurayama-shi Tokyo 189-0022 (JP); DOI, Takeshi, Higashiyamato-shi Tokyo 207-0011 (JP)
(74) Representative: Hartz, Nikolai
(86) International application number: PCT/JP2007/001107
(87) International publication number: WO 2008/044337

(57) **Abstract**

The present invention is directed to a γ-globin inducer, to a prophylactic and/or therapeutic agent for sickle cell disease, and to a prophylactic and/or therapeutic agent for β-thalassemia, each containing, as an active ingredient, 4-[N-(4-methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine, a salt thereof, or a solvate of either of these.

## Description

### Technical Field

The present invention relates to a novel γ-globin inducer and to a prophylactic/therapeutic agent for a disease caused by production of mutant β-globin; e.g., sickle cell disease or β-thalassemia.

### Background Art

Hemoglobin, which is a protein that transports oxygen molecules to various tissues, is a tetramer formed of two kinds of polypeptides. In an early stage of development, hemoglobin is formed of two ε chains and two ζ chains (ε2ζ2), but during a development stage is replaced by fetal hemoglobin (HbF) formed of two α chains and two γ chains (α2γ2) through switching in transcription of a hemoglobin gene. In a prenatal stage, HbF undergoes gene switching to transform adult hemoglobin (HbA) formed of two α chains and two β chains (α2β2). As a result, in the hemoglobin composition of healthy adults' erythrocytes, HbA cells account for 97% with the remainder (3%) being composed mainly of HbA2 (α2δ2).

Meanwhile, sickle cell disease(hereinafter referred to as SCD) is known as one of diseases related to abnormality in hemoglobin. Specifically, SCD is a hereditary disease caused by mutation of the sixth amino acid residue of a β-chain gene forming HbA from glutamine to valine, producing abnormal hemoglobin S (HbS). In most cases, sickle homozygotes die by adulthood, whereas sickle heterozygotes develop embolus-related symptoms including hematuria. The onset mechanism of SCD includes polymerization of HbS molecules under low-oxygen conditions, gelation caused by a decrease in solubility, formation of sickle cells, selective decay in the spleen, and occurrence of emboli in organs such as the heart, lungs, liver, and brain, inducing various systemic symptoms.

Another known hereditary disease is thalassemia, which is caused by hereditarily abnormal hemoglobin, resulting in hypochromic anemia. Particularly, a type of thalassemia including impairment in production of normal β-globin caused by a defect in a structural gene of a hemoglobin β chain is called β-thalassemia. In β-thalassemia, abnormality is induced, in organs such as the stomach, liver, and kidneys, by anemia caused by erythrocytes having shortened lifetime, intake of an excessive amount of iron by frequent transfusion, and other causes. In grave cases, patients die from heart failure.

Currently, therapeutic means for these diseases is limited to removal of the spleen, transfusion, administration of iron-chelating agent, etc. However, such therapeutic means are problematic in terms of cumbersome therapeutic operations, cost, adverse side effects, etc. Thus, there is keen demand for development of effective therapeutic methods therefor.
One possible approach is administration, to such a patient, of an erythropoietin (EPO) formulation or a drug for promoting production of EPO, which are generally employed in the treatment of anemia. In this case, since production of adult hemoglobin is induced, and abnormal hemoglobin merely increases. Thus, this approach cannot ameliorate the symptoms.

Since SCD and β-thalassemia are diseases caused by a defect of a gene of hemoglobin β chain, induction of expressing an endogenous γ-chain gene to thereby increase HbF (α2γ2) having normal oxygen transport function is thought to be effective for treatment of such diseases.

Hitherto, there have been known a variety of compounds which induce expression of a γ-globin gene, but a more effective compound is expected.
Among such known compounds, hydroxyurea increases a globin gene, but the effect is not specific to the γ-chain gene (Non-Patent Document 1). Some butyric acid derivatives are reported to increase to a γ chain specifically, but pharmacokinetic characteristics are not satisfactory and a large amount administration thereof is required (Non-Patent Documents 2 and 3). Cytosine arabinoside and 5-azacytidine are known to have γ-globin gene expression inducing function, but these compounds are chemotherapeutic agents having cytotoxicity, which imposes limitation on use thereof (Non-Patent Document 4). Also reported is that a low-oxygen inducing factor-proline hydroxydase inhibitor induces γ-globin in cultured cells (Patent Document 1). Dilazep hydrochloride is known to be useful as a therapeutic agent for thalassemia and sickle cell anemia, but whether or not the compound promotes production of γ-globin has never been disclosed (Patent Documents 2 and 3).

As disclosed in Patent Document 4, a known compound, 4-[N-(4-methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine, has an excellent cell adhesion inhibitory action and cellular infiltration inhibitory action and is applied to allergic diseases, autoimmune diseases, and chronic inflammatory diseases. Other disclosed uses of the compound include anti-cancer action (Patent Document 5), erythropoietin production promoting action (Patent Document 6), and vascularization inhibitory action (Patent Document 7). However, these documents never disclose whether or not the compound has γ-globin gene expression inducing action.
[Patent Document 1] WO 2005/011696
[Patent Document 2] JP-A-1990-202820
[Patent Document 3] JP-A-1983-96019
[Patent Document 4] WO 03/020703
[Patent Document 5] WO 03/086397
[Patent Document 6] WO 2004/052859
[Patent Document 7] WO 2005/034953
[Non-Patent Document 1] Lettvin et al., N. Engl. J. Med. Vol. 310, p.p. 869-873 (1984)
[Non-Patent Document 2] Dover et al., Blood, Vol. 84, No. 1, p.p. 339-343 (1994)
[Non-Patent Document 3] Collins et al., Blood; Vol. 85, No. 1, p.p. 43-49 (1995)
[Non-Patent Document 4] Desimoine et al., Blood, Vol. 99, No. 1, p.p. 3905-3908 (2002)

### Disclosure of the Invention

### Problems to be Solved by the Invention

An object of the present invention is to provide a novel γ-globin inducer. Another object of the present invention is to provide a pharmaceutical agent useful for preventing or treating a disease caused by production of mutant β-globin; e.g., sickle cell disease or β-thalassemia. Means for Solving the Problems

Under such circumstances, the present inventors have conducted extensive studies and have found that 4-[N-(4-methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine has an excellent γ-globin gene expression inducing action and an excellent hemoglobin production promoting action and therefore serves as a useful prophylactic or therapeutic agent for a disease caused by production of mutant β-globin. The present invention has been accomplished on the basis of this finding.

Accordingly, the present invention provides a γ-globin inducer containing, as an active ingredient, 4-[N-(4-methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine, an acid-added salt thereof, or a solvate of either of these.

The present invention also provides a prophylactic and/or therapeutic agent for sickle cell disease containing, as an active ingredient, 4-[N-(4-methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine, an acid-added salt thereof, or a solvate of either of these.

The present invention also provides a prophylactic and/or therapeutic agent for β-thalassemia containing, as an active ingredient, 4-[N-(4-methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine, an acid-added salt thereof, or a solvate of either of these.

The present invention also provides use of 4-[N-(4-methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine, an acid-added salt thereof, or a solvate of either of these, for producing a γ-globin inducer, a prophylactic and/or therapeutic agent for sickle cell disease, or a prophylactic and/or therapeutic agent for β-thalassemia.

The present invention also provides a method for preventing and/or treating a disease caused by production of mutant β-globin, characterized by administering, to a subject in need thereof, an effective amount of 4-[N-(4-methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine, an acid-added salt thereof, or a solvate of either of these.

### Effects of the Invention

4-[N-(4-Methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine has an excellent γ-globin gene expression inducing action. Therefore, these species are useful γ-globin inducers or prophylactic and/or therapeuctic agents for a disease caused by production of mutant β-globin, such as sickle cell disease or β-thalassemia.

### Brief Description of the Drawings

[Fig. 1] A graph showing the effect of compound 1 on production of hemoglobin in K562 cells.
[Fig. 2] A graph showing the effect of compound 1 on expression of γ-globin mRNA in K562 cells.
[Fig. 3] A graph showing induction of HbF-producing cells in K562 cells by compound 1.

### Modes for Carrying Out the Invention

4-[N-(4-Methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine (hereinafter may be referred to as compound 1), which is an active ingredient of the present invention, and a pharmaceutical product containing compound 1 as an active ingredient may be produced by the method disclosed in the pamphlet of International Publication WO 2003/02703.

In the present invention, compound 1, an acid-added salt thereof, or a solvate of the acid-added salt may be employed. Examples of the acid of the acid-added salt include inorganic acids such as sulfuric acid, hydrochloric acid, nitric acid, phosphoric acid, and hydrobromic acid; and organic acids such as acetic acid, lactic acid, succinic acid, tartaric acid, malic acid, maleic acid, citric acid, fumaric acid, methanesulfonic acid, and toluenesulfonic acid. Such a salt may be a solvate, typically a hydrate. The present invention also encompasses polymorphism of compound 1, an acid-added salt of compound 1, and a solvate of thereof.

The prophylactic and/or therapeutic agent of the present invention contains, as an active ingredient, compound 1, a salt thereof, or a solvate of either of these. No particular limitation is imposed on the administration form, and any of the drug forms, for example, oral drugs, injection solutions, suppositories, ointments, inhalations, eye drops, nasal drops, and adhesive preparations, may be selected in accordance with the purpose of the treatment. Compositions suitable for the administration forms may be produced through mixing the active ingredient with a pharmaceutically acceptable carrier on the basis of a drug preparation method generally known in the art.

The oral solid pharmaceutical product may be prepared by mixing compound 1 with a vehicle and an optional binder, disintegrant, lubricant, coloring agent, flavoring agent, odor improving agent, etc., and forming the mixture into tablets, coated-tablets, granules, powder, capsules, etc. through a method known in the art. These additives may be those generally employed in the art. Examples of the vehicle include lactose, sucrose, sodium chloride, glucose, starch, calcium carbonate, kaolin, microcrystalline cellulose, and silicic acid. Examples of the binder include water, ethanol, propanol, simple syrup, liquid glucose, liquid starch, liquid gelatin, carboxymethyl cellulose, hydroxypropyl cellulose, hydroxypropyl starch, methyl cellulose, ethyl cellulose, shellac, calcium phosphate, and polyvinylpyrrolidone. Examples of the disintegrant include dry starch, sodium alginate, agar powder, sodium hydrogencarbonate, calcium carbonate, sodium lauryl sulfate, monoglyceryl stearate, and lactose. Examples of the lubricant include refined talc, stearate salts, borax, and polyethylene glycol. Examples of the flavoring agent include sucrose, orange peel, citric acid, and tartaric acid.

The oral liquid pharmaceutical product may be prepared by mixing the aforementioned compound with a flavoring agent, buffer, stabilizer, odor improving agent, etc. and forming the mixture into internal liquid, syrup, elixir, etc. through a method known in the art. The flavoring agent employed in the preparation may be any of the aforementioned members. Examples of the buffer include sodium citrate. Examples of the stabilizer include traganth, acacia, and gelatin.

The injection solutions may be prepared by mixing compound 1 with additives such as a pH-regulator, buffer, stabilizer, tonicity agent, and local anesthetic agent, and mixing the mixture through a method known in the art, to thereby provide subcutaneous, intramuscular, and intravenous injection liquids. Example of the pH-regulator and buffer include sodium citrate, sodium acetate, and sodium phosphate. Examples of the stabilizer include sodium pyrosulfite, EDTA, thioglycolic acid, and thiolactic acid. Examples of the local anesthetic agent include procaine hydrochloride and lidocaine hydrochloride. Examples of the tonicity agent include sodium chloride and glucose.

The suppositories may be prepared by mixing compound 1 with a carrier for pharmaceutical production known in the art such as polyethylene glycol, lanolin, cacao butter, and fatty acid triglyceride, and with an optional surfactant such as Tween (registared trademark), and forming the mixture into suppositories through a method known in the art.

The ointments may be prepared by mixing compound 1 with optional additives generally employed in the art such as a base, stabilizer, moisturizer, and preservatives, and forming the mixture into ointments through a method known in the art. Examples of the base include liquid paraffin, white petrolatum, white beeswax, octyldodecyl alcohol, and paraffin. Examples of the preservative include methyl p-hydroxybenzoate, ethyl p-hydroxybenzoate, and propyl p-hydroxybenzoate.

The pharmaceutical product of the present invention containing compound 1 may be formed into inhalations, eye drops, or nasal drops, through a method known in the art.

The daily dose of the pharmaceutical agent of the present invention, which varies depending on the age, sex, body weight, symptom, administration route, and time of administration of a patient, is generally 0.01 to 1,000 mg as compound 1 for an adult, preferably 0.1 to 100 mg. Preferably, the pharmaceutical agent is orally or parenterally administered to a patient singly or in a divided manner.

The thus-produced prophylactic and/or therapeutic agent serves as an excellent γ-globin inducer, and thus is a useful prophylactic and/or therapeutic agent for a disease caused by production of mutant β-globin such as sickle cell disease and β-thalassemia.

### Examples

The present invention will next be described in more detail by way of examples, which should not be construed as limiting the scope of the invention thereto.

### Example 1

### A. Materials and method

### 1) Compound employed

Compound 1 was synthesized through a method disclosed in International publication WO 03/02703 (Example 13) and dissolved in dimethyl sulfoxide (DMSO). The concentration of the solution was adjusted as appropriate through dilution. An equiamount of DMSO was employed as a control solution.

### 2) Cell culture

Cells of human proerythroblast cell line K562 (obtained from ATCC) were added to a complete culture medium (RPMI-1640 medium containing 10% fetal bovine serum), and the resultant cells (1 × 10⁵ cells/mL) were seeded into each well of 24-well plate (2 mL/well). A solution of compound 1 (×1000) was added to the plate (2 µL/well), and cells were cultured in a CO₂ incubator (37°C, 5%CO₂) for three days. The medium was renewed, and cells were further cultured for three days.

### 3) Measurement of the amount of hemoglobin

The cultured cells were collected and counted. The number of cells in each sample was adjusted to 3 × 10⁵, and the intensity of the fluorescence attributed to a porphyrin ring was measured, to thereby determine the hemoglobin level. Specifically, cells collected through centrifugation were suspended in 2M oxalic acid (500 µL), and the suspension was boiled for 30 minutes and cooled. Fluorescence intensity was measured by means of a fluorescence microplate reader (Spectra MAX Gemini EM, product of Molecular Device) (Em: 400 nm, Ex: 603 nm).

### B. Results

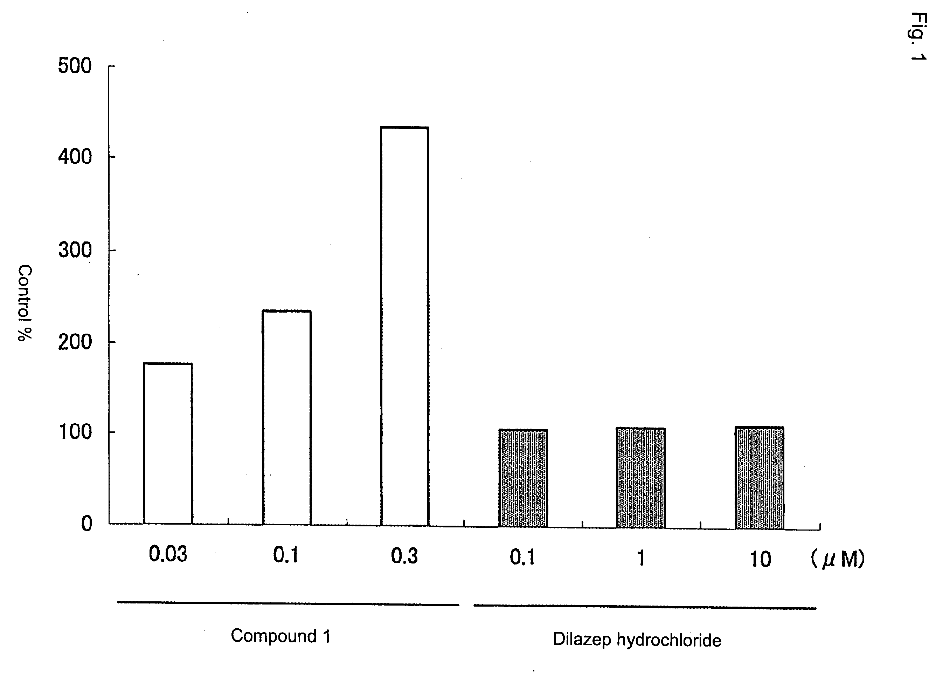

Fig. 1 shows the effect of compound 1 on the amount of hemoglobin produced by K562 cells. In Fig. 1, the amount of hemoglobin produced in a compound-non-added group is represented by 100%. As is clear from Fig. 1, compound 1 promotes production of hemoglobin in a concentration (dose)-dependent manner. In a 0.3-µM-added group, the amount reached 435%. In contrast, dilazep hydrochloride, which has a sickling inhibitory action and is known to exhibit a thalassemia therapeutic effect based on an inhibitory action on promotion of platelet aggregation, did not exhibit hemoglobin production promoting action.

### Example 2

### A. Method

K562 cells (1 × 10⁵ cells/mL) were seeded into each well of 6-well plate (4 mL/well). A solution of compound 1 (×1000) was added to the plate (4 µL/well), and cells were cultured in a CO₂ incubator (37°C, 5%CO₂) for three days. The cultured cells were collected, and RNA was prepared from the cells by means of a RNeasy mini column (product of Qiagen). Subsequently, cDNA was synthesized from the thus-obtained RNA by use of reverse transcriptase. Through real-time PCR employing a TaqMan probe according to a protocol of Applied Bio-Systems, γ-globin mRNA level was determined (ABI PRISM7900HT System, product of Applied Bio-Systems). In Example 2, the following primers and probe were employed (SEQ ID NOs: 1 to 3):
forward primer (GGTTCTTTGACAGCTTTG, SEQ ID NO: 1);
reverse primer (CCTTCTTGCCATGTGCCTT, SEQ ID NO: 2); and
fluorescence probe (CCTCTGCCTCTGCCATC, SEQ ID NO: 3).
These primers and probe are custom synthesis products of Qiagen.

### B. Results

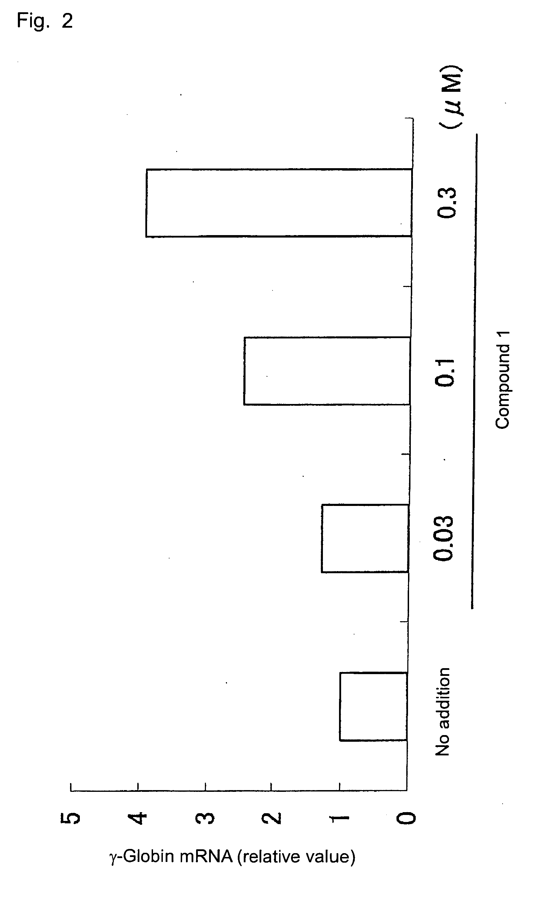

Fig. 2 shows the effect of compound 1 on the amount of γ-globin mRNA produced by K562 cells. In Fig. 2, the amount of γ-globin mRNA produced in a compound-non-added group is represented by 1. As is clear from Fig. 2, compound 1 increased the amount of γ-globin mRNA produced in a 0.3-µM-added group by about four fold in a concentration (dose)-dependent manner.

### Example 3

### A. Method

K562 cells (1 × 10⁵ cells/mL) were seeded into each well of 24-well plate (2 mL/well). A solution of compound 1 (×1000) was added to the plate (2 µL/well), and cells were cultured in a CO₂ incubator (37°C, 5%CO₂) for three days. The cultured cells were collected through centrifugation and treated for 10 minutes with phosphate buffered saline (PBS) containing 0.05% glutaraldehyde/0.1% bovine serum albumin (BSA). The liquid was removed from the mixture through centrifugation, to thereby obtain pellets. Subsequently, 0.1% BSA/PBS was added to the separated pellets, and the same treatment was performed three times, to thereby wash the cells. The thus-washed cells were subjected, for five minutes, to permeation treatment with 0.1% BSA/PBS containing 1% Triton X-100 (0.5 mL) and washed once again. The resultant cells were suspended in 0.1% BSA/PBS (80 µL). FITC-labeled anti-HbF antibody (10 µg/mL, product of Caltag) (5 µL) was added to the suspension, and the mixture was allowed to react at room temperature in the dark for 15 minutes, whereby HbF present in the cells was immunostained. In a similar manner as described above, the stained cells were washed and suspended in 0.1% BSA/PBS containing 1% formaldehyde (0.5 mL). The suspension was analyzed through a method known in the art, by means of a flow cytometer (EPICS XL, product of Beckman Coulter, Inc.).

### B. Results

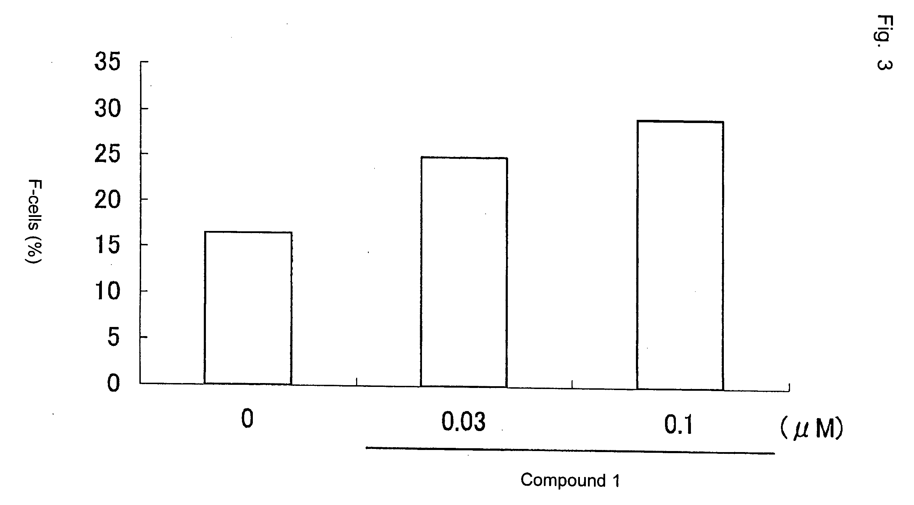

Fig. 3 shows the percentage of HbF-producing cells (F cells) contained in the K562 cell population. As is clear from Fig. 3, the number of HbF-expressing cells was almost doubled through addition of 0.1-µM compound 1.

Through the aforementioned pharmacological tests, compound 1 has been found to have a hemoglobin production promoting action. In particular, a strong γ-globin inducing action was observed even at a small dose of the compound. Therefore, compound 1 of the present invention, a salt thereof, or a solvate of either of these has been found to serve as an excellent γ-globin inducer.

As described hereinabove, a pharmaceutical agent containing, as an active ingredient, compound 1 of the present invention, a salt thereof, or a solvate of either of these is useful for prevention of onset or progress of a disease caused by production of mutant β-globin; e.g., sickle cell disease or β-thalassemia, amelioration of pathological conditions of the disease, treatment of the disease, etc.

## Claims

1. A γ-globin inducer containing, as an active ingredient, 4-[N-(4-methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine, an acid-added salt thereof, or a solvate of either of these.

2. A prophylactic and/or therapeutic agent for sickle cell disease containing, as an active ingredient, 4-[N-(4-methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine, an acid-added salt thereof, or a solvate of either of these.

3. A prophylactic and/or therapeutic agent for β-thalassemia containing, as an active ingredient, 4-[N-(4-methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine, an acid-added salt thereof, or a solvate of either of these.

4. Use of 4-[N-(4-methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine, an acid-added salt thereof, or a solvate of either of these, for producing a γ-globin inducer.

5. Use of 4-[N-(4-methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine, an acid-added salt thereof, or a solvate of either of these, for producing a prophylactic and/or therapeutic agent for sickle cell disease.

6. Use of 4-[N-(4-methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine, an acid-added salt thereof, or a solvate of either of these, for producing a prophylactic and/or therapeutic agent for β-thalassemia.

7. A method for preventing and/or treating a disease caused by production of mutant β-globin, wherein the method comprises administering, to a subject in need thereof, an effective amount of 4-[N-(4-methoxyphenyl)-N-[[5-(3,4,5-trimethoxyphenyl)pyridin-3-yl]methyl]amino]-1-[[2-(3,4,5-trimethoxyphenyl)pyridin-4-yl]methyl]piperidine, an acid-added salt thereof, or a solvate of either of these.


SEQUENCE LISTING
<110> KOWA CO., LTD.
<120> gamma-globin inducer
<130> KW0203
<150> JP2006-279893
<151> 2006-10-13
<160> 3
<170> PatentIn Ver. 2.1
<210> 1
<211> 18
<212> DNA
<213> Artificial sequence
<220>
<223> Designed primer based on human gamma-globin mRNA sequence
<400> 1
ggttctttga cagctttg                                18
<210> 2
<211> 19
<212> DNA
<213> Artificial sequence

<220>

<223> Designed primer based on human gamma-globin mRNA sequence

<400> 2

ccttcttgcc atgtgcctt                                    19

<210> 3

<211> 17

<212> DNA

<213> Artificial sequence

<220>

<223> Designed fluorophore-probe based on human gamma-globin mRNA sequence

<400> 3

cctctgcctc tgccatc                                      17